(19) Europäisches Patentamt
European Patent Office
Office européen des brevets

(11) EP 2 813 483 A1

(12) EUROPEAN PATENT APPLICATION
published in accordance with Art. 153(4) EPC

(43) Date of publication:
**17.12.2014 Bulletin 2014/51**

(21) Application number: **13746041.6**

(22) Date of filing: **04.02.2013**

(51) Int Cl.:
***C07C 43/13*** *(2006.01)*
***A61K 8/34*** *(2006.01)*
***A61Q 1/14*** *(2006.01)*
***C07C 43/178*** *(2006.01)*
***C08G 65/28*** *(2006.01)*

(86) International application number:
**PCT/JP2013/052461**

(87) International publication number:
**WO 2013/118676 (15.08.2013 Gazette 2013/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority:
**08.02.2012 JP 2012024654**
**22.03.2012 JP 2012065439**
**27.03.2012 JP 2012070869**

(71) Applicant: **Daicel Corporation**
**Osaka-shi, Osaka 530-0001 (JP)**

(72) Inventors:
- **MAEHARA, Tetsuya**
  **Ohtake-shi**
  **Hiroshima 739-0695 (JP)**
- **SAKANISHI, Yuichi**
  **Ohtake-shi**
  **Hiroshima 739-0695 (JP)**
- **ABURANO, Daisuke**
  **Himeji-shi**
  **Hyogo 671-1283 (JP)**
- **KITAO, Kyuhei**
  **Himeji-shi**
  **Hyogo 671-1283 (JP)**

(74) Representative: **Grünecker, Kinkeldey, Stockmair & Schwanhäusser**
**Leopoldstrasse 4**
**80802 München (DE)**

# (54) POLYGLYCERIN DIALKYL OR ALKENYL ETHER, AND COSMETIC COMPOSITION CONTAINING SAME

(57) Provided are: a novel polyglycerol di-(alkyl/alkenyl) ether that is useful as a highly hydrophilic gemini surfactant; and a cosmetic composition containing the polyglycerol di-(alkyl/alkenyl) ether. The polyglycerol di-(alkyl/alkenyl) ether is represented by Formula (1), where $R^1$ and $R^2$ are identical or different and are independently a straight- or branched-chain alkyl group or a straight- or branched-chain alkenyl group, where the alkyl group may have one or more hydroxyl groups; and n indicates a number of glycerol units and is an integer of 2 or more. Formula (1) is expressed as follows:

[Chem. 1]

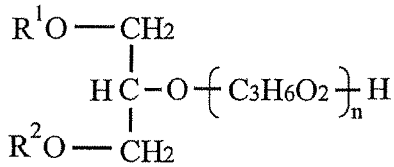

(1)

EP 2 813 483 A1

## Description

Technical Field

The present invention relates to novel polyglycerol di-(alkyl/alkenyl) ethers; and cosmetic compositions containing the same. The polyglycerol di-(alkyl/alkenyl) ethers are useful as highly hydrophilic gemini surfactants.

Background Art

Cleansing cosmetics for use in the area of cosmetics to remove a makeup stain come in various forms. Typically, such cleansing cosmetics include those containing large amounts of oils (oil components) and available typically in the form of creams, milky lotions, oils, and oily gels; and those containing none or trace amounts of oils and available typically in the form of lotions and aqueous gels. The cleansing cosmetics containing large amounts of oils offer excellent detergency (cleansing power), but are disadvantageously inferior in usability. Typically, such oil-based cleansing cosmetics offer a sticky feel, fail to be easily rinsed away with water, and need another cleansing typically with a facial cleansing agent. In addition, it is difficult to use them in a bath, because they are hardly mixed with a makeup stain when the skin is wet. In contrast, the cleansing cosmetics containing none or trace amounts of oils offer good usability, but disadvantageously have low detergency.

An aqueous gel cleansing cosmetic has been reported as a cleansing agent having both detergency and usability (Patent Literature (PTL) 1). The aqueous gel cleansing cosmetic contains a polyoxyethylene fatty acid ester; one of a polyhydric alcohol and a glycol ether; and a water-soluble polymer compound. The polyoxyethylene fatty acid ester is one of monomeric surfactants, i.e., surfactants each having one hydrophobic group and one hydrophilic group. The aqueous gel cleansing cosmetic is, however, still insufficient in detergency.

In contrast, a gemini surfactant is a compound geometrically including two molecules of monomeric surfactant bonded to each other via a short spacer. The gemini surfactant, as having the structure, can be intimately adsorbed at the interface between an aqueous phase and an oil phase without repulsion of the two molecules bonded via a short spacer. For this reason, it is known that the gemini surfactant can be used in a smaller amount less than or equal to half the amount of a conventional equivalent (monomeric surfactant) and is environmentally friendly. This is because, when a monomeric surfactant is compared with a corresponding gemini surfactant in half the number of moles of the monomeric surfactant, the gemini surfactant advantageously offers a higher surface activity (detergency) than the monomeric surfactant, although the two surfactants have a hydrophilic group and a hydrophobic group in identical numbers to each other. It is also known that the gemini surfactant has a low Krafft point and can exhibit excellent solubility and solubilizing power even at low temperatures.

Exemplary known gemini surfactants having excellent performance as mentioned above include one synthesized from oleic acid; and one extracted from a natural product. However, the synthesis from oleic acid and the extraction from a natural product require complicated processes and suffer from low yields. Thus, the gemini surfactants are disadvantageously very expensive. In addition, there is little knowledge at present on gemini surfactants having high hydrophilicity, although gemini surfactants having high hydrophobicity have been increasingly investigated (PTL 2, PTL 3, and PTL 4).

Citation List

Patent Literature

PTL 1: Japanese Unexamined Patent Application Publication (JP-A) No. H08-283123
PTL 2: JP-A No. S59-105073
PTL 3: JP-A No. S64-9304
PTL 4: JP-A No. 2001-354998

Summary of Invention

Technical Problem

Accordingly, an object of the present invention is to provide a novel polyglycerol di-(alkyl/alkenyl) ether that is useful as a highly hydrophilic gemini surfactant.

Another object of the present invention is to provide a cosmetic composition containing the polyglycerol

di-(alkyl/alkenyl) ether.

Yet another object of the present invention is to provide a cleansing cosmetic composition having both detergency and usability, i.e., a cleansing cosmetic composition which is readily miscible with a makeup stain regardless of whether the skin is wet or not, which can exhibit excellent detergency, and which can be washed away cleanly without leaving an oily feel by rinsing with water.

Still another object of the present invention is to provide a cleansing cosmetic containing the cleansing cosmetic composition.

Solution to Problem

After intensive investigations to achieve the objects, the present inventors have found a compound that is obtained by allowing an alcohol to react with a glycidyl ether to give a glycerol di-(alkyl/alkenyl) ether having a secondary hydroxyl group, and allowing the glycerol di-(alkyl/alkenyl) ether to further react with 2 equivalents or more of glycidol; and that the compound, as including two hydrophobic groups bonded to each other via a short spacer and having a highly water-soluble polyglycerol moiety as a hydrophilic group, offers an excellent surface activity and has a high HLB value (having high hydrophilicity).

The present inventors have also found that the compound can exhibit an excellent surface activity even in a small amount and, when added to a cosmetic, can exhibit excellent osmotic, emulsifying, and dispersing actions while much less causing irritation to the skin; and that a cleansing cosmetic containing the compound is readily miscible with a makeup stain, regardless of whether the skin is wet or not, and can exhibit excellent detergency ; and that the cleansing cosmetic can be washed away cleanly without leaving an oily feel by rinsing with water. The present invention has been made based on these findings and further investigations.

Specifically, the present invention provides, in an embodiment, a polyglycerol di-(alkyl/alkenyl) ether represented by Formula (1) expressed as follows:

[Chem. 1]

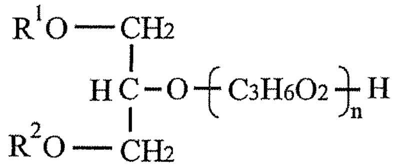

(1)

where $R^1$ and $R^2$ are identical or different and are independently one of a straight- or branched-chain alkyl group and a straight- or branched-chain alkenyl group, where the alkyl group may have one or more hydroxyl groups; and n indicates a number of glycerol units and is an integer of 2 or more.

In the polyglycerol di-(alkyl/alkenyl) ether, $R^1$ and $R^2$ may be identical or different and may are independently one of a straight- or branched-chain alkyl group having 6 to 22 carbon atoms and a straight- or branched-chain alkenyl group having 6 to 22 carbon atoms, where the alkyl group may have one or more hydroxyl groups; and n is an integer of from 2 to 25.

The present invention provides, in another embodiment, a cosmetic composition containing the polyglycerol di-(alkyl/alkenyl) ether.

The present invention provides, in still another embodiment, a cleansing cosmetic composition containing 1 to 70 percent by weight of the polyglycerol di-(alkyl/alkenyl) ether and 30 to 99 percent by weight of water.

In addition and advantageously, the present invention provides a cleansing cosmetic containing the cleansing cosmetic composition.

The cleansing cosmetic may further contain a polymer compound.

Advantageous Effects of Invention

The polyglycerol di-(alkyl/alkenyl) ether according to the embodiment of the present invention, as having the configuration, can offer an extremely excellent surface activity. The polyglycerol di-(alkyl/alkenyl) ether according to the embodiment of the present invention, when used as a surfactant, can therefore exhibit sufficient effects even in a smaller amount than those of conventional equivalents and can contribute to significant reduction of environmental load and skin irritation. The polyglycerol di-(alkyl/alkenyl) ether according to the embodiment of the present invention, as having the configuration, has a low Krafft point, can exhibit excellent solubility and solubilizing power even at low temperatures, and, when used typically for cleansing, can be completely washed away even with water. The polyglycerol di-(alkyl/alkenyl) ether according to the embodiment of the present invention is advantageously usable typically in cosmetic compositions such as hair cosmetic compositions (typically for hair conditioners), skin cosmetic compositions (typically for skin care lotions), and cleansing cosmetic compositions (typically for makeup cosmetic cleansing agents); detergent compositions; solubilizers; dispersing agents; emulsifiers; wetting agents; dyestuffs; antimicrobial agents; antistatic agents; and spreaders.

The cleansing cosmetic composition according to the embodiment of the present invention, as containing the polyglycerol di-(alkyl/alkenyl) ether, offers a light feel of use without stickiness. The cleansing cosmetic composition is readily miscible with a makeup stain, regardless of whether the skin is wet or not, can exhibit excellent detergency, and can be washed away cleanly without leaving an oily feel by rinsing with water. The cleansing cosmetic composition according to the embodiment of the present invention is advantageously usable in or as cleansing agents for removing makeup cosmetics. Brief Description of Drawings

[Fig. 1] Fig. 1 depicts $^1$H-NMR (400 MHz, $CDCl_3$) spectral data of a compound obtained in Example 3.
[Fig. 2] Fig. 2 depicts $^1$H-NMR (400 MHz, $CDCl_3$) spectral data of a compound obtained in Example 4.
[Fig. 3] Fig. 3 depicts $^1$H-NMR (400 MHz, DMSO) spectral data of a compound obtained in Example 6.
[Fig. 4] Fig. 4 depicts $^1$H-NMR (400 MHz, DMSO) spectral data of a compound obtained in Example 7.

Description of Embodiments

Polyglycerol Di-(alkyl/alkenyl) Ether

The polyglycerol di-(alkyl/alkenyl) ether according to the embodiment of the present invention is represented by Formula (1). In Formula (1), $R^1$ and $R^2$ are identical or different and are independently one of a straight- or branched-chain alkyl group and a straight- or branched-chain alkenyl group, where the alkyl group may have one or more hydroxyl groups. The number n indicates a number of glycerol units and is an integer of 2 or more. Formula (1) is expressed as follows:

[Chem. 2]

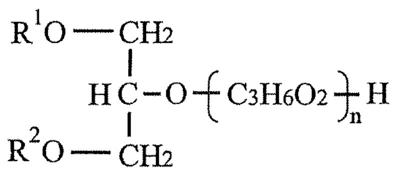

(1)

The moiety $C_3H_6O_2$ in the brackets in Formula (1) can assume either of two structures represented by Formulae (2) and (3) expressed as follows:

$-CH_2-CHOH-CH_2O-$ (2)

$-CH(CH_2OH)CH_2O-$ (3)

The straight- or branched-chain alkyl group as $R^1$ and $R^2$ is exemplified by straight chain alkyl groups having 6 to 22 carbon atoms, such as n-hexyl, n-heptyl, n-octyl, n-nonyl, n-decyl, n-undecyl, n-lauryl, n-tridecyl, n-tetradecyl (myristyl), n-pentadecyl, n-hexadecyl, n-heptadecyl, n-stearyl, n-nonadecyl, n-eicosyl, n-heneicosyl, and n-docosyl groups; and branched chain alkyl groups having 6 to 22 carbon atoms, such as isohexyl, s-hexyl, t-hexyl, isoheptyl, s-heptyl, t-heptyl, isooctyl, s-octyl, t-octyl, isononyl, s-nonyl, t-nonyl, isodecyl, s-decyl, t-decyl, isoundecyl, s-undecyl, t-undecyl, isolauryl, s-lauryl, t-lauryl, isotridecyl, s-tridecyl, t-tridecyl, isotetradecyl, s-tetradecyl, t-tetradecyl, isopentadecyl, s-pentadecyl, t-pentadecyl, isocetyl, isohexadecyl, s-hexadecyl, t-hexadecyl, isoheptadecyl, s-heptadecyl, t-heptadecyl, isostearyl, isononadecyl, s-nonadecyl, t-nonadecyl, isoeicosyl, s-eicosyl, t-eicosyl, isohenicosyl, s-henicosyl, t-henicosyl, isodocosyl, s-docosyl, and t-docosyl groups.

The straight- or branched-chain alkyl group having one or more hydroxyl groups as $R^1$ and $R^2$ is exemplified by straight- or branched-chain hydroxyalkyl groups having 6 to 22 carbon atoms, corresponding to the straight- or branched-chain alkyl groups having 6 to 22 carbon atoms, except for having one or more hydroxyl groups.

The straight- or branched-chain alkenyl group as $R^1$ and $R^2$ is exemplified by straight chain alkenyl groups having 6 to 22 carbon atoms, such as n-hexenyl, n-heptenyl, n-octenyl, n-nonenyl, n-decenyl, n-undecenyl, n-dodecenyl,

n-tridecenyl, n-tetradecenyl, n-pentadecenyl, n-hexadecenyl, n-heptadecenyl, n-oleyl, n-nonadecenyl, n-eicosenyl, n-heneicosenyl, and n-docosenyl groups; and branched chain alkenyl groups having 6 to 22 carbon atoms, such as isohexenyl, s-hexenyl, t-hexenyl, isoheptenyl, s-heptenyl, t-heptenyl, isooctenyl, s-octenyl, t-octenyl, isononenyl, s-nonenyl, t-nonenyl, isodecenyl, s-decenyl, t-decenyl, isoundecenyl, s-undecenyl, t-undecenyl, isododecenyl, s-dodecenyl, t-dodecenyl, isotridecenyl, s-tridecenyl, t-tridecenyl, isotetradecenyl, s-tetradecenyl, t-tetradecenyl, isopentadecenyl, s-pentadecenyl, t-pentadecenyl, isohexadecenyl, s-hexadecenyl, t-hexadecenyl, isoheptadecenyl, s-heptadecenyl, t-heptadecenyl, isooleyl, isononadecenyl, s-nonadecenyl, t-nonadecenyl, isoeicosenyl, s-eicosenyl, t-eicosenyl, isohenicosenyl, s-henicosenyl, t-henicosenyl, isodocosenyl, s-docosenyl, and t-docosenyl groups.

Preferred as $R^1$ and $R^2$ herein are straight- or branched-chain alkyl or alkenyl groups having 8 to 20 (more preferably 8 to 18, furthermore preferably 10 to 18, particularly preferably 10 to 16, and most preferably 10 to 14) carbon atoms, of which straight chain alkyl groups are more preferred. This is because the resulting polyglycerol dialkyl ether can form a firm palisade layer at the interface and can thereby form a lamellar liquid crystalline phase.

The number n indicates the number of glycerol units, is an integer of 2 or more, and may be typically from 2 to 25, preferably from 2 to 15, and particularly preferably from 2 to 10. The polyglycerol di-(alkyl/alkenyl) ether, if having a number "n" less than the range, may tend to have a lower HLB value (have lower hydrophilicity) and to offer lower solubility in an aqueous component. In contrast, the polyglycerol di-(alkyl/alkenyl) ether, if having an excessively large number "n", may tend to have an excessively high HLB value (have lower lipophilicity) and to offer lower solubility in an oily component. When used particularly in a detergent composition, the polyglycerol di-(alkyl/alkenyl) ether preferably has a number "n" of from 2 to 20, particularly preferably from 2 to 12, and most preferably from 2 to 10. This is preferred for excellent detergency and foaming power. When used particularly in a cosmetic composition (e.g., a cleansing cosmetic composition), the polyglycerol di-(alkyl/alkenyl) ether has a number "n" of preferably from 3 to 10, more preferably from 5 to 10, particularly preferably from 7 to 10, and most preferably from 8 to 10. This is preferred because the resulting polyglycerol di-(alkyl/alkenyl) ether offers extremely low irritation to the skin and excels in foaming power (particularly foaming power under wet conditions) and a feel of use.

The polyglycerol di-(alkyl/alkenyl) ether according to the embodiment of the present invention has a hydrophile-lipophile balance (HLB) of typically 5.0 or more, preferably from 5.5 to 15.0, and particularly preferably from 6.0 to 15.0. The polyglycerol di-(alkyl/alkenyl) ether, if having a HLB less than the range, may often offer lower hydrophilicity and lower solubility in an aqueous component. The HLB value may be determined typically by calculation according to an after-mentioned expression using an organic conceptual diagram. Regarding physicochemical properties of a compound, the degree of property caused mainly by Van Der Waals force is referred to as "organicity"; whereas the degree of property caused mainly by electric affinity is referred to as "inorganicity". A "value of organicity (organic value)" and a "value of inorganicity (inorganic value)" can be calculated as a sum of values of individual structural moieties typically based on the compound structure ("Kaimenkasseizai no Gosei to sono Oyo" (in Japanese; "Syntheses and Applications of Surfactants"), Ryohei ODA and Kazuhiro TERAMURA, Maki Shoten, Tokyo, March 1957). A computational expression for the calculation of the HLB value is not limited to the expression as follows:

$$\text{HLB value} = [(\text{Inorganicity})/(\text{Organicity})] \times 10$$

The polyglycerol di-(alkyl/alkenyl) ether according to the embodiment of the present invention is exemplified by triglycerol dioctyl ether, triglycerol didecyl ether, triglycerol dilauryl ether, triglycerol ditetradecyl ether, triglycerol dioleyl ether, triglycerol distearyl ether, triglycerol diisostearyl ether, tetraglycerol dioctyl ether, tetraglycerol didecyl ether, tetraglycerol dilauryl ether, tetraglycerol ditetradecyl ether, tetraglycerol dioleyl ether, tetraglycerol distearyl ether, tetraglycerol diisostearyl ether, pentaglycerol dioctyl ether, pentaglycerol didecyl ether, pentaglycerol dilauryl ether, pentaglycerol ditetradecyl ether, pentaglycerol dioleyl ether, pentaglycerol distearyl ether, pentaglycerol diisostearyl ether, hexaglycerol dioctyl ether, hexaglycerol didecyl ether, hexaglycerol dilauryl ether, hexaglycerol ditetradecyl ether, hexaglycerol dioleyl ether, hexaglycerol distearyl ether, hexaglycerol diisostearyl ether, heptaglycerol dioctyl ether, heptaglycerol didecyl ether, heptaglycerol dilauryl ether, heptaglycerol ditetradecyl ether, heptaglycerol dioleyl ether, heptaglycerol distearyl ether, heptaglycerol diisostearyl ether, octaglycerol dioctyl ether, octaglycerol didecyl ether, octaglycerol dilauryl ether, octaglycerol ditetradecyl ether, octaglycerol dioleyl ether, octaglycerol distearyl ether, octaglycerol diisostearyl ether, nonaglycerol dioctyl ether, nonaglycerol didecyl ether, nonaglycerol dilauryl ether, nonaglycerol ditetradecyl ether, nonaglycerol dioleyl ether, nonaglycerol distearyl ether, nonaglycerol diisostearyl ether, decaglycerol dioctyl ether, decaglycerol didecyl ether, decaglycerol dilauryl ether, decaglycerol ditetradecyl ether, decaglycerol dioleyl ether, decaglycerol distearyl ether, decaglycerol diisostearyl ether, undecaglycerol dioctyl ether, undecaglycerol didecyl ether, undecaglycerol dilauryl ether, undecaglycerol ditetradecyl ether, undecaglycerol dioleyl ether, undecaglycerol distearyl ether, and undecaglycerol diisostearyl ether.

The polyglycerol di-(alkyl/alkenyl) ether according to the embodiment of the present invention structurally has

two hydrophobic groups ($R^1$ and $R^2$) bonded to each other via a short spacer, can be intimately adsorbed at an interface without repulsion, and can thereby offer an extremely excellent surface activity. The polyglycerol di-(alkyl/alkenyl) ether can therefore be used in an amount less than or equal to half the amount of a conventional equivalent, applies a smaller load on the environment, and causes much less irritation typically to the skin. The polyglycerol di-(alkyl/alkenyl) ether according to the embodiment of the present invention has high solubility in water and has a low Krafft point (typically 10°C or lower, and preferably from 0°C to 5°C) because a secondary hydroxyl group of the polyglycerol moiety serving as a hydrophilic group forms a hydrogen bond with water. The polyglycerol di-(alkyl/alkenyl) ether can therefore exhibit excellent solubility and solubilizing power even at low temperatures.

The polyglycerol di-(alkyl/alkenyl) ether according to the embodiment of the present invention, as having the characteristic properties, is advantageously usable typically in cosmetic compositions such as hair cosmetic compositions (typically for hair conditioners), skin cosmetic compositions (typically for skin care lotions), and cleansing cosmetic compositions (typically for cleansing agents for makeup cosmetics); detergent compositions; solubilizers; dispersing agents; emulsifiers; wetting agents; dyestuffs; antimicrobial agents; antistatic agents; and spreaders.

Polyglycerol Di-(alkyl/alkenyl) Ether Production Method The polyglycerol di-(alkyl/alkenyl) ether represented by Formula (1) may be produced typically by a method of allowing an alkyl or alkenyl glycidyl ether to react with an aliphatic alcohol in the presence of a catalyst to give a glycerol di-(alkyl/alkenyl) ether and allowing the resulting glycerol di-(alkyl/alkenyl) ether to further react with glycidol.

More specifically, the polyglycerol di-(alkyl/alkenyl) ether represented by Formula (1) may be produced typically through Steps (1) and (2) as expressed by an after-mentioned scheme. In the scheme, $R^1$, $R^2$, and n are as defined above.

Step (1): An alcohol represented by Formula (4) is allowed to react with a glycidyl ether represented by Formula (5) to give a glycerol di-(alkyl/alkenyl) ether of Formula (6) having a secondary hydroxyl group; and

Step (2): The glycerol di-(alkyl/alkenyl) ether of Formula (6) having a secondary hydroxyl group as obtained from Step (1) is allowed to react with glycidol, where glycidol is used in an amount of "n" equivalents per one equivalent of the glycerol di-(alkyl/alkenyl) ether, and Formulae (4), (5), and (6) are expressed as follows:

[Chem. 3]

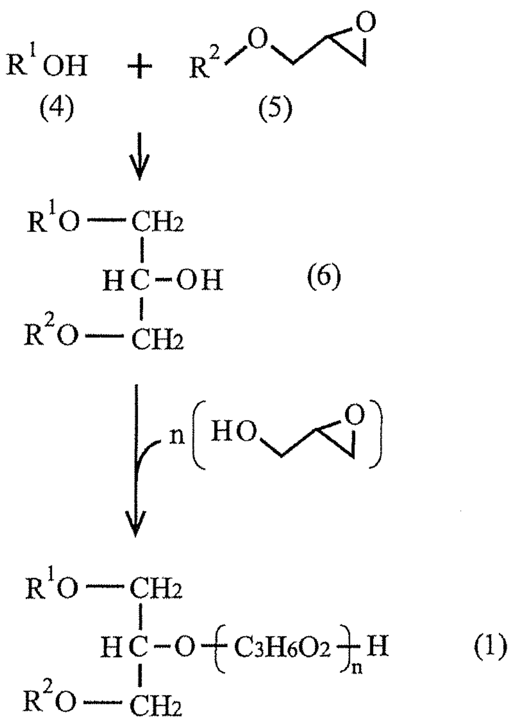

(4) (5) (6) (1)

Step (1) also gives a by-product glycerol di-(alkyl/alkenyl) ether represented by Formula (6') expressed as follows:

[Chem. 4]

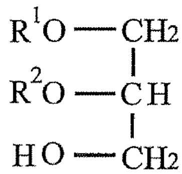

(6')

In Step (2), this compound gives a polyglycerol di-(alkyl/alkenyl) ether represented by Formula (1') expressed as follows:

[Chem. 5]

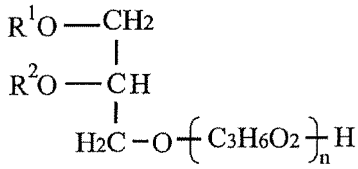

(1')

A byproduct ratio of the polyglycerol di-(alkyl/alkenyl) ether represented by Formula (1') is desirably 20% or less, preferably 15% or less, and particularly preferably 10% or less. $R^1$, $R^2$, and n in Formulae (1') and (6') are as defined above. The byproduct ratio of the polyglycerol di-(alkyl/alkenyl) ether represented by Formula (1') may be adapted typically by controlling the catalyst amount and/or the reaction temperature.

$R^1$ in the alcohol represented by Formula (4) corresponds to $R^1$ in the polyglycerol di-(alkyl/alkenyl) ether represented by Formula (1) and is one selected from a straight- or branched-chain alkyl group and a straight- or branched-chain alkenyl group, where the alkyl group may have one or more hydroxyl groups. $R^1$ herein is preferably a straight- or branched-chain alkyl or alkenyl group having 8 to 20 (more preferably 8 to 18, furthermore preferably 10 to 18, particularly preferably 10 to 16, and most preferably 10 to 14) carbon atoms, of which a straight chain alkyl group is more preferred.

The alcohol represented by Formula (4) is exemplified by saturated alcohols such as decyl alcohol, undecyl alcohol, lauryl alcohol, tridecyl alcohol, tetradecyl alcohol, stearyl alcohol, and isostearyl alcohol; and unsaturated alcohols such as oleyl alcohol, isooleyl alcohol, and linolyl alcohol (i.e., linolenyl alcohol). Each of them may be used alone or in combination.

$R^2$ in the glycidyl ether represented by Formula (5) corresponds to $R^2$ in the polyglycerol di-(alkyl/alkenyl) ether represented by Formula (1) and is one selected from a straight- or branched-chain alkyl group and a straight- or branched-chain alkenyl group, where the alkyl group may have one or more hydroxyl groups. $R^2$ herein is preferably a straight- or branched-chain alkyl or alkenyl group having 8 to 20 (more preferably 8 to 18, furthermore preferably 10 to 18, particularly preferably 10 to 16, and most preferably 10 to 14) carbon atoms, of which a straight chain alkyl group is more preferred.

The glycidyl ether represented by Formula (5) is exemplified by glycidyl ethers having a straight- or branched-chain alkyl group as $R^2$, such as decyl glycidyl ether, undecyl glycidyl ether, lauryl glycidyl ether, tridecyl glycidyl ether, tetradecyl glycidyl ether, stearyl glycidyl ether, and isostearyl glycidyl ether; and glycidyl ethers having a straight- or branched-chain alkenyl group as $R^2$, such as oleyl glycidyl ether, isooleyl glycidyl ether, and linolyl glycidyl ether (i.e., linolenyl glycidyl ether). Each of them may be used alone or in combination.

The glycidyl ether represented by Formula (5) may be used in the reaction in Step (1) in an amount of typically from about 3 to about 10 equivalents and preferably from 6 to 8 equivalents per 1 equivalent of the alcohol represented by Formula (4). The glycidyl ether represented by Formula (5), if used in an amount out of the range, may readily cause the polyglycerol di-(alkyl/alkenyl) ether represented by Formula (1) to be formed in a lower yield.

The reaction in Step (1) is preferably performed in the presence of an acid catalyst. The acid catalyst can be either of a protonic acid and a Lewis acid. The protonic acid is exemplified by organic acids and inorganic acids, including super strong acids (such as $SbF_5$, $SbF_5$-HF, $SbF_5$-$FSO_3H$, and $SbF_5$-$CF_3SO_3H$), sulfuric acid, hydrochloric acid, phosphoric acid, fluoroboric acid, p-toluenesulfonic acid, chloroacetic acid, picric acid, and heteropolyacids. The Lewis acid is exemplified by $BF_3$, $BF_3O(C_2H_5)_2$, $AlCl_3$, $FeCl_3$, tin alkoxides (such as tin tetraisopropoxide), tin halides (such as tin tetrachloride (i.e., tin(IV) chloride) and tin tetrabromide).

The acid catalyst may be used in an amount of typically from about 0.5 to about 6 mole percent and preferably

from 1 to 5 mole percent relative to the alcohol represented by Formula (4). The acid catalyst, if used in an amount greater than the range, may accelerate the formation of the by-product and may often cause the polyglycerol di-(alkyl/alkenyl) ether represented by Formula (1) to be formed with a lower purity. In contrast, the acid catalyst, if used in an amount less then the range, may often cause the polyglycerol di-(alkyl/alkenyl) ether represented by Formula (1) to be formed in a lower yield.

The reaction in Step (1) may be performed at a temperature of typically from about 50°C to about 150°C and preferably from 60°C to 100°C for a time of typically from about 30 minutes to about 5 hours and preferably from 30 minutes to 2 hours.

The reaction in Step (1) may be performed in any atmosphere not limited, such as air atmosphere, nitrogen atmosphere, or argon atmosphere, as long as not adversely affecting the reaction. The reaction can also be performed according to any system such as a batch system, semi-batch system, or continuous system.

After the completion of reaction, a reaction product may be separated and purified by a separation procedure such as filtration, concentration, distillation, or extraction, or a separation procedure as a combination of them.

The reaction in Step (2) is preferably performed in the presence of a basic catalyst.

The basic catalyst is exemplified by alkali metal hydroxides such as lithium hydroxide, sodium hydroxide, potassium hydroxide, and cesium hydroxide; alkali metal carbonates such as lithium carbonate, sodium carbonate, potassium carbonate, and cesium carbonate; alkali metal hydrogencarbonates such as sodium hydrogencarbonate, potassium hydrogencarbonate, and cesium hydrogencarbonate; alkaline earth metal hydroxides such as magnesium hydroxide, calcium hydroxide, and barium hydroxide; alkaline earth metal carbonates such as magnesium carbonate, calcium carbonate, and barium carbonate; alkali metal alkoxides such as sodium methoxide, sodium ethoxide, and potassium t-butoxide; alkali metal organic acid salts such as sodium acetate; and amines (e.g., tertiary amines) and nitrogen-containing heterocyclic compounds, such as triethylamine, piperidine, N-methylpiperidine, and pyridine.

Among them, an alkali metal alkoxide such as sodium methoxide is preferably employed as the base catalyst for keeping production cost low.

The basic catalyst may be used in an amount of typically from about 20 to about 100 mole percent and preferably from 90 to 100 mole percent relative to the glycerol di-(alkyl/alkenyl) ether of Formula (6) having a secondary hydroxyl group. The basic catalyst, if used in an amount greater than the range, may readily accelerate the formation of the by-product polyglycerol. In contrast, the basic catalyst, if used in an amount less than the range, may often cause the glycerol di-(alkyl/alkenyl) ether of Formula (6) having a secondary hydroxyl group to remain unreacted.

The reaction in Step (2) may be performed in any atmosphere not limited, such as air atmosphere, nitrogen atmosphere, or argon atmosphere, as long as not adversely affecting the reaction. The reaction can be performed according to any system such as a batch system, semi-batch system, or continuous system.

The reaction in Step (2) may be performed at a temperature of typically from about 50°C to about 150°C and preferably from 60°C to 120°C for a time of typically from about 30 minutes to about 24 hours, preferably from 5 hours to 15 hours, and particularly preferably from 7 hours to 12 hours. The reaction in Step (2) can be terminated by the addition typically of an aqueous phosphoric acid solution, sulfuric acid, hydrochloric acid, or acetic acid.

After the completion of reaction, a reaction product may be separated and purified by a separation procedure such as filtration, concentration, distillation, or extraction, or a separation procedure as a combination of them.

Cleansing Cosmetic Composition

The cleansing cosmetic composition according to the embodiment of the present invention contains the polyglycerol di-(alkyl/alkenyl) ether and water.

The cleansing cosmetic composition according to the embodiment of the present invention may contain the polyglycerol di-(alkyl/alkenyl) ether in a content of typically from about 1 to about 70 percent by weight based on the total amount (100 percent by weight) of the composition. When the cleansing cosmetic composition contains two or more different polyglycerol di-(alkyl/alkenyl) ethers, the "content" refers to a total content of them. An upper limit of the polyglycerol di-(alkyl/alkenyl) ether content is preferably 50 percent by weight, particularly preferably 30 percent by weight, and most preferably 20 percent by weight. A lower limit thereof is preferably 5 percent by weight. The composition, if containing the polyglycerol di-(alkyl/alkenyl) ether in a content less than the range, may readily offer lower detergency. In contrast, the composition, if containing the polyglycerol di-(alkyl/alkenyl) ether in a content greater than the range, may have a higher viscosity and may readily offer inferior handleability.

The water may be any of a hard water and a soft water and can be selected typically from industrial water, service water (tap water), ion-exchanged water, or distilled water.

The cleansing cosmetic composition according to the embodiment of the present invention may contain water in a content of typically from about 30 to about 99 percent by weight based on the total amount (100 percent by weight) of the composition. An upper limit of the water content is preferably 50 percent by weight, particularly preferably 60 percent by weight, and most preferably 80 percent by weight; whereas an upper limit thereof is preferably 95 percent

by weight and particularly preferably 93 percent by weight. The composition, if containing water in a content less than the range, may readily offer lower usability. In contrast, the composition, if containing water in a content greater than the range, may readily offer lower detergency.

The cleansing cosmetic composition according to the embodiment of the present invention may be prepared typically by mixing and stirring the components at an ambient temperature of from 40°C to 80°C.

The cleansing cosmetic composition according to the embodiment of the present invention, when diluted ten times with water, may have a pH of typically from about 4 to about 10 and preferably from 5 to 9.

Cleansing Cosmetic

The cleansing cosmetic according to the embodiment of the present invention contains the cleansing cosmetic composition. The cleansing cosmetic according to the embodiment of the present invention may be in any form such as a lotion, solution, milky lotion, cream, or gel form.

The cleansing cosmetic according to the embodiment of the present invention may further contain one or more other components in addition to the cleansing cosmetic composition. In a preferred embodiment of the present invention, the cleansing cosmetic contains a polymer compound for a better feel of use and better usability.

The polymer compound (macromolecular compound) is preferably a water-soluble polymer compound which is exemplified by plant-derived natural polymer compounds such as carrageenan and guar gum; microbial or microbe-mediated natural polymer compounds such as xanthan gum; animal-derived natural polymer compounds such as casein and gelatin; cellulosic polymer compounds such as carboxymethylcellulose, methylcellulose, ethylcellulose, and hydroxyethylcellulose; starch polymer compounds such as carboxy-methyl starch; vinyl polymer compounds such as poly(vinyl alcohol)s, polyvinylpyrrolidones, sodium polyacrylate, and carboxyvinyl polymers; and polyether polymer compounds such as polyethylene glycols. Each of them may be used alone or in combination.

Among them, preferred as the polymer compound for use herein are polyether polymer compounds such as polyethylene glycols; plant-derived natural polymer compounds such as carrageenan; vinyl polymer compounds such as carboxyvinyl polymers; and cellulosic polymer compounds such as hydroxyethylcellulose.

The cleansing cosmetic may contain the polymer compound in an amount of typically from about 0.1 to about 50 percent by weight based on the total amount (100 percent by weight) of the cleansing cosmetic. When the cleansing cosmetic contains two or more different polymer compounds, the "amount" refers to a total amount of them. An upper limit of the polymer compound amount is preferably 10 percent by weight and particularly preferably 3 percent by weight. A lower limit thereof is preferably 0.2 percent by weight and particularly preferably 0.3 percent by weight.

The cleansing cosmetic according to the embodiment of the present invention may further contain any of other components according to necessity in addition to the aforementioned components, within ranges achieving the objects of the present invention. Such other components are exemplified by nonionic surfactants other than the polyglycerol di-(alkyl/alkenyl) ethers according to the embodiment of the present invention, anionic surfactants, amphoteric surfactants, alcohols, powder components, antioxidants, antioxidation assistants, ultraviolet absorbers, humectants (moisturizers), anti-inflammatory agents, antiseptic agents, pH adjusters, extracts (extracts from animals, plants, fishes and shellfishes, or microbes), and flavors. Each of them may be used alone or in combination.

The nonionic surfactants other than the polyglycerol di-(alkyl/alkenyl) ethers according to the embodiment of the present invention are exemplified by surfactants having no ionizable group as a hydrophilic group, such as glycerol fatty acid esters, polyglycerol fatty acid esters, polyalkylene glycol fatty acid esters, sorbitan fatty acid esters, sugar fatty acid esters, pentaerythritol fatty acid esters, polyoxyalkylene hydrogenated castor oil fatty acid esters, fatty acid alkanolamides, polyoxyalkylene glycols, esters between a polyoxyalkylene glycol and a monohydric or polyhydric alcohol, polyoxyalkylene sugar ethers, condensates between a fatty amide and a polyoxyalkylene glycol, condensates between an aliphatic amine and a polyoxyalkylene glycol, and alkyl or alkenyl polyglycosides.

The anionic surfactants are exemplified by polyoxyethylene alkyl ether sulfates, alkyl sulfate salts, alkylbenzenesulfonates, $\alpha$-olefinsulfonates, amino acid surfactants (e.g., glutamic acid), N-acyl-methyl-taurates, and alkyl phosphate salts.

The amphoteric surfactants are exemplified by carboxybetaine, imidazolinium, sulfobetaine, and alanine amphoteric surfactants.

The alcohols are exemplified by lower alcohols such as ethanol, propyl alcohol, ethylene glycol, and diethylene glycol.

The powder components include inorganic powders and organic powders. The inorganic powders are exemplified by talc, kaolin, mica, sericite, muscovite, phlogopite, synthetic mica, lepidolite, biotite, lithia mica, vermiculite, magnesium carbonate, zirconium silicate, aluminum silicate, barium silicate, calcium silicate, zinc silicate, magnesium silicate, strontium silicate, metal tungstates, magnesium, silica, zeolite, barium sulfate, calcium sulfate hemihydrate (calcined gypsum), calcium phosphate, fluoroapatite, hydroxyapatite, ceramic powders, activated carbon, medical carbon, metal soaps (e.g., zinc myristate, calcium palmitate, and aluminum stearate), and boron nitride. The organic powders

are exemplified by polyamide resin powders (nylon powders), polyethylene powders, poly(methyl methacrylate) powders, polystyrene powders, powders of copolymers between styrene and acrylic acid, benzoguanamine resin powders, and cellulose powders.

The antioxidants are exemplified by vitamin E, dibutylhydroxytoluene, butylhydroxyanisole, and gallic acid esters.

The antioxidation assistants are exemplified by ascorbic acid, phytic acid, kephalin, and maleic acid.

The ultraviolet absorbers are exemplified by benzophenone derivatives such as 2-hydroxy-4-methoxybenzophenone, 2-hydroxy-4-methoxybenzophenone-5-sulfonic acid and salts thereof, and dihydroxydimethoxybenzophenone; p-aminobenzoic acid, and derivatives thereof such as p-aminoethyl benzoate; p-methoxycinnamic acid derivatives such as ethyl p-methoxycinnamate, isopropyl p-methoxycinnamate, octyl p-methoxycinnamate, and methoxycinnamic acid derivatives; salicylic acid derivatives such as octyl salicylate and phenyl salicylate; urocanic acid and derivatives thereof; 4-tert-butyl-4'-methoxydibenzoylmethane; 2-(2'-hydroxy-5'-methylphenyl)benzotriazole; and methyl anthranilate.

The humectants are exemplified by glycerol, propylene glycol, 1,3-butylene glycol, sorbitol, sodium lactate, and pyrrolidonecarboxylic acid and salts thereof.

The anti-inflammatory agents are exemplified by glycyrrhizic acid and derivatives thereof, glycyrrhetic acid and derivatives thereof, allantoin, hydrocortisone acetate, and azulene.

The antiseptic agents are exemplified by methylparaben, propylparaben, and phenoxyethanol.

The pH adjusters are exemplified by citric acid, hydrochloric acid, sulfuric acid, phosphoric acid, sodium hydroxide, and ammonia.

The extracts (extracts from animals, plants, fishes/shellfishes, or microbes) are exemplified by tea extract, aloe extract, Ginkgo biloba extract, swertia herb extract, mugwort extract, Allium sativum (garlic) extract, Scutellaria baicalensis root extract, Rosmarinus officinalis (rosemary) extract, Luffa cylindrica extract, placental extract, extract from lactic acid bacteria culture, and seaweed extract.

The cleansing cosmetic according to the embodiment of the present invention may be prepared typically by mixing and stirring the cleansing cosmetic composition and optional other component(s) (e.g., a polymer compound).

The cleansing cosmetic according to the embodiment of the present invention, as having the configuration, has safety, a light feel of use, and an excellent surface activity. The cleansing cosmetic according to the embodiment of the present invention is readily miscible with a makeup stain, regardless of whether the skin is wet or not, can exhibit excellent detergency, and is usable even in a bath. The cleansing cosmetic can wash a makeup stain cleanly by rinsing with water. The cleansing cosmetic according to the embodiment of the present invention is advantageously usable as a cleansing agent typically for a makeup cosmetic.

## Examples

The present invention will be illustrated in further detail with reference to several examples below. It should be noted, however, that the examples are by no means intended to limit the scope of the invention.

### Production Example 1: Production of didecylglycerol

Decyl alcohol (a reagent supplied by Wako Pure Chemical Industries, Ltd., 886 g) was charged in an amount of 8 equivalents per 1 equivalent of after-mentioned decyl glycidyl ether. In a nitrogen atmosphere, 1 percent by mole tin(IV) chloride (1.82 g) was added, and decyl glycidyl ether (supplied by Yokkaichi Chemical Co., Ltd., 150 g) was added dropwise over one hour while maintaining the reaction temperature at 80°C. After the completion of dropwise addition, the mixture was aged for one hour, and water was added to terminate the reaction.

The resulting crude reaction mixture was dehydrated by adding potassium carbonate (supplied by Wako Pure Chemical Industries, Ltd., 9.67 g), followed by dilution with heptane (supplied by Wako Pure Chemical Industries, Ltd., 259 g).

The diluted mixture was subjected to suction filtration using a Buchner funnel, the reactor was rinsed with a small amount of heptane, and a filtrate was obtained.

Heptane was distilled off from the filtrate on an evaporator, the remainder was subjected to distillation at 150°C at a reduced pressure of 2 mmHg to distill off unreacted decyl glycidyl ether and thereby yielded 372 g of didecylglycerol.

### Production Example 2: Production of dilauryl glycerol

Dilaurylglycerol was obtained by the procedure of Production Example 1, except for using lauryl glycidyl ether and lauryl alcohol instead of decyl glycidyl ether and decyl alcohol, respectively.

Production Example 3: Production of ditetradecyl glycerol

Ditetradecylglycerol was obtained by the procedure of Production Example 1, except for using tetradecyl glycidyl ether and tetradecyl alcohol instead of decyl glycidyl ether and decyl alcohol, respectively.

Production Example 4: Production of dioleyl glycerol

Dioleylglycerol was obtained by the procedure of Production Example 1, except for using oleyl glycidyl ether and oleyl alcohol instead of decyl glycidyl ether and decyl alcohol, respectively.

Example 1: Production of triglycerol dilauryl ether

After distilling off lauryl glycidyl ether at 200°C and 2 mmHg, 104 g of dilaurylglycerol obtained in Production Example 2 was combined with a 28% sodium methoxide methanol solution (46.9 g), from which methanol was distilled off by distillation at 100°C and a reduced pressure of 2 mmHg for 10 hours.

Glycidol (54.0 g) was then added dropwise in an amount of 2 equivalents per 1 equivalent of the dilaurylglycerol over 10 hours while maintaining the temperature at 70°C. After aging for one hour, 18.7 g of a 85% aqueous phosphoric acid solution was added to terminate the reaction.

The resulting crude reaction mixture was diluted with 150 g of methanol.

From the crude mixture, a phosphoric acid salt was removed by pressure filtration; methanol was distilled off at 150°C and normal atmospheric pressure; other low-boiling components were distilled off at 2 mmHg; and thereby yielded 158 g of triglycerol dilauryl ether (HLB: 6.33).

Example 2: Production of triglycerol ditetradecyl ether

Triglycerol ditetradecyl ether (HLB: 5.14) was obtained by the procedure of Example 1, except for using ditetradecylglycerol obtained in Production Example 3 instead of dilaurylglycerol obtained in Production Example 2.

Example 3: Production of tetraglycerol dilauryl ether

Tetraglycerol dilauryl ether (HLB: 6.94) was obtained by the procedure of Example 1, except for using glycidol in an amount of 3 equivalents instead of 2 equivalents. Fig. 1 depicts $^1$H-NMR (400 MHz, $CDCl_3$) spectral data of the obtained compound.

Example 4: Production of tetraglycerol didecyl ether

Tetraglycerol didecyl ether (HLB: 6.87) was obtained by the procedure of Example 1, except for using didecylglycerol obtained in Production Example 1 instead of dilaurylglycerol obtained in Production Example 2; and using glycidol in an amount of 3 equivalents instead of 2 equivalents. Fig. 2 depicts $^1$H-NMR (400 MHz, $CDCl_3$) spectral data of the obtained compound.

Example 5: Production of tetraglycerol dioleyl ether

Tetraglycerol dioleyl ether (HLB: 5.25) was obtained by the procedure of Example 1, except for using dioleylglycerol obtained in Production Example 4 instead of dilaurylglycerol obtained in Production Example 2; and using glycidol in an amount of 3 equivalents instead of 2 equivalents.

Example 6: Production of pentaglycerol didecyl ether

Pentaglycerol didecyl ether (HLB: 8.86) was obtained by the procedure of Example 1, except for using didecylglycerol obtained in Production Example 1 instead of dilaurylglycerol obtained in Production Example 2; and using glycidol in an amount of 4 equivalents instead of 2 equivalents. Fig. 3 depicts $^1$H-NMR (400 MHz, DMSO) spectral data of the obtained compound.

Example 7: Production of pentaglycerol dilauryl ether

Pentaglycerol dilauryl ether (HLB: 10.0) was obtained by the procedure of Example 1, except for using glycidol in an amount of 4 equivalents instead of 2 equivalents. Fig. 4 depicts $^1$H-NMR (400 MHz, DMSO) spectral data of the

obtained compound.

Example 8: Production of heptaglycerol didecyl ether

Heptaglycerol didecyl ether (HLB: 10.5) was obtained by the procedure of Example 1, except for using didecylglycerol obtained in Production Example 1 instead of dilaurylglycerol obtained in Production Example 2; and using glycidol in an amount of 6 equivalents instead of 2 equivalents.

Example 9: Production of heptaglycerol dilauryl ether

Heptaglycerol dilauryl ether (HLB: 11.3) was obtained by the procedure of Example 1, except for using glycidol in an amount of 6 equivalents instead of 2 equivalents.

Example 10: Production of heptaglycerol ditetradecyl ether

Heptaglycerol ditetradecyl ether (HLB: 8.78) was obtained by the procedure of Example 1, except for using ditetradecylglycerol obtained in Production Example 3 instead of dilaurylglycerol obtained in Production Example 2; and using glycidol in an amount of 6 equivalents instead of 2 equivalents.

Example 11: Production of nonaglycerol dilauryl ether

Nonaglycerol dilauryl ether (HLB: 12.3) was obtained by the procedure of Example 1, except for using glycidol in an amount of 8 equivalents instead of 2 equivalents.

Example 12: Production of decaglycerol ditetradecyl ether

Decaglycerol ditetradecyl ether (HLB: 12.2) was obtained by the procedure of Example 1, except for using ditetradecylglycerol obtained in Production Example 3 instead of dilaurylglycerol obtained in Production Example 2; and using glycidol in an amount of 9 equivalents instead of 2 equivalents.

Example 13: Production of undecaglycerol dilauryl ether

Undecaglycerol dilauryl ether (HLB: 13.2) was obtained by the procedure of Example 1, except for using glycidol in an amount of 10 equivalents instead of 2 equivalents.

Example 14: Production of undecaglycerol ditetradecyl ether

Undecaglycerol ditetradecyl ether (HLB: 11.0) was obtained by the procedure of Example 1, except for using ditetradecylglycerol obtained Production Example 3 instead of dilaurylglycerol obtained in Production Example 2; and using glycidol in an amount of 10 equivalents instead of 2 equivalents.

Example 15: Production of undecaglycerol dioleyl ether

Undecaglycerol dioleyl ether (HLB: 9.74) was obtained by the procedure of Example 1, except for using dioleylglycerol obtained in Production Example 4 instead of dilaurylglycerol obtained in Production Example 2; and using glycidol in an amount of 10 equivalents instead of 2 equivalents.

Example 16: Production of henicosaglycerol dilauryl ether

Henicosaglycerol dilauryl ether (HLB: 14.6) was obtained by the procedure of Example 1, except for using glycidol in an amount of 20 equivalents instead of 2 equivalents.

Examples 17 to 21

Detergent compositions were prepared by blending materials in percentages given in Table 1 below. Surfactants used herein were the compounds obtained in the examples above.

The resulting detergent compositions were examined to evaluate detergency and foaming power by test methods as follows.

Detergency Test

An artificial stain was attached to a flat 20-$cm^2$ area glass plate, the glass plate was set on a dedicated platform, immersed in 700 mL of a 0.2% aqueous solution of a sample detergent composition at a liquid temperature of 30°C, and subjected to cleaning by rotating impellers at number of revolutions of 250 rpm for 3 minutes.
The glass plate was retrieved after the completion of rotation, dried, weighed, from which a cleaning ratio was calculated according to an expression as follows, and the detergency was evaluated according to criteria as follows.

$$\text{Cleaning ratio}=\{[(\text{Weight before cleansing})-(\text{Weight after cleansing})]/(\text{Weight before cleansing})\}\times 100\%$$

Criteria:

A: detergency of 95% or more;
B: detergency of from 85% to less than 95%;
C: detergency of 50% to less than 85%; and
F: detergency of less than 50%

Foaming Power Test

A sample detergent composition was diluted 20 folds with water to give a dilute aqueous solution, and 100 mL of the solution at a liquid temperature of 30°C was charged into a 500-mL graduated cylinder. Next, impellers were placed in the solution and rotated at a number of revolutions of 1000 rpm for one minute to stirrer the solution. The volume (mL) of foam formed after the process was measured and defined as a foaming quantity. The foaming power was evaluated based on the foaming quantity according to criteria as follows.

Criteria

A: foaming quantity of 300 mL or more;
B: foaming quantity of from 250 mL to less than 300 mL;
C: foaming quantity of from 200 mL to less than 250 mL; and
F: foaming quantity of less than 200 mL

The results are catalogued in a table as follows.

[Table 1]

TABLE 1

| Detergent composition | | Example | | | | |
|---|---|---|---|---|---|---|
| | | 17 | 18 | 19 | 20 | 21 |
| Surfactant | Triglycerol ditetradecyl ether | 20.0 | | | | |
| | Tetraglycerol dilauryl ether | | 20.0 | | | |
| | Pentaglycerol dilauryl ether | | | 20.0 | | |
| | Decaglycerol ditetradecyl ether | | | | 20.0 | |
| | Henicosaglycerol dilauryl ether | | | | | 20.0 |
| Water | | 80.0 | 80.0 | 80.0 | 80.0 | 80.0 |

(continued)

| Detergent composition | | Example | | | | |
|---|---|---|---|---|---|---|
| | | 17 | 18 | 19 | 20 | 21 |
| Evaluation | Detergency | B | B | A | B | B |
| | Foaming power | B | B | B | B | B |

Table 1 demonstrates that the polyglycerol di-(alkyl/alkenyl) ethers according to the embodiment of the present invention offered excellent detergency and foaming power.

Examples 22 to 31 and Comparative Examples 1 to 10

Cleansing cosmetics were obtained by blending materials in percentages (percent by weight) as given in Tables 2 and 3 below and stirring them at 60°C. Surfactants used herein were the compounds obtained in the examples above.

The obtained cleansing cosmetics were evaluated by methods as follows.

Detergency Test and Feel of Use Evaluation

1. Detergency Test under Dry Condition

A lipstick (trade name "MAQuillAGE Superior Rouge RD759", supplied by Shiseido Co., Ltd.) (0.2 g) was applied to the forearm; and about 0.5 g of each of the cleansing cosmetics obtained in the examples and comparative examples was taken in the hand and sufficiently mixed with the lipstick by massaging the applied portion ten times. The applied portion was washed (rinsed) with water; how the lipstick was removed was visually observed; and the detergency was evaluated according to criteria below. Skin feel after cleansing (washing) was organoleptically evaluated according to criteria below.

2. Detergency Test under Wet Condition with Water

A lipstick (trade name "MAQuillAGE Superior Rouge RD759", supplied by Shiseido Co., Ltd.) (0.2 g) was applied to the forearm; the forearm was wetted with water; about 0.5 g of each of the cleansing cosmetics obtained in the examples and comparative examples was taken in the hand and sufficiently mixed with the lipstick by massaging the applied portion ten times. The lipstick was then washed with water, how the lipstick was removed was visually observed, and the detergency was evaluated according to criteria as follows:

Detergency Criteria:

A: lipstick was completely removed;
B: lipstick was nearly removed;
C: lipstick remained a little; and
F: lipstick was hardly removed

Feel of Use Criteria:

A: very light feel;
B: light feel;
C: somewhat oily feel (slimy feel); and
F: oily feel

The results are catalogued in tables as follows.

[Table 2]

TABLE 2

| Cleansing cosmetic | | | Example | | | | | | | | | |
|---|---|---|---|---|---|---|---|---|---|---|---|---|
| | | | 22 | 23 | 24 | 25 | 26 | 27 | 28 | 29 | 30 | 31 |
| Water | | | 89.5 | 89.5 | 89.5 | 89.5 | 89.5 | 89.5 | 89.5 | 89.5 | 89.5 | 89.5 |
| Surfactant | Undecaglycerol dilauryl ether | | 10.0 | | | | | 10.0 | | | | |
| | Nonaglycerol dilauryl ether | | | 10.0 | | | | | 10.0 | | | |
| | Heptaglycerol dilauryl ether | | | | 10.0 | | | | | 10.0 | | |
| | Pentaglycerol dilauryl ether | | | | | 10.0 | | | | | 10.0 | |
| | Tetraglycerol dilauryl ether | | | | | | 10.0 | | | | | 10.0 |
| Polymer compound | Polyethylene glycol | | 0.5 | 0.5 | 0.5 | 0.5 | 0.5 | | | | | |
| | Carboxyvinyl polymer | | | | | | | 0.5 | 0.5 | 0.5 | 0.5 | 0.5 |
| Evaluation | Detergency | Wet condition | A | A | A | B | B | A | A | A | B | B |
| | | Non-wet condition | A | A | A | A | A | A | A | A | A | A |
| | Feel of use | | A | A | B | B | B | A | A | B | B | B |

[Table 3]

TABLE 3

| Cleansing cosmetic | | | Comparative Example | | | | | | | | | |
|---|---|---|---|---|---|---|---|---|---|---|---|---|
| | | | 1 | 2 | 3 | 4 | 5 | 6 | 7 | 8 | 9 | 10 |
| Water | | | 89.5 | 89.5 | 89.5 | 89.5 | 89.5 | 89.5 | 89.5 | 89.5 | 89.5 | 89.5 |
| Surfactant | Polyethylene glycol monolaurate | | 10.0 | | | | | 10.0 | | | | |
| | Polyoxyethylene sorbitan monoisostearyl ether | | | 10.0 | | | | | 10.0 | | | |
| | Polyoxyethylene sorbitan coconut fatty acid ester | | | | 10.0 | | | | | 10.0 | | |
| | Polyoxyethylene hydrogenated castor oil | | | | | 10.0 | | | | | 10.0 | |
| | Polyoxyethylene octyldodecyl ether | | | | | | 10.0 | | | | | 10.0 |
| Polymer compound | Polyethylene glycol | | 0.5 | 0.5 | 0.5 | 0.5 | 0.5 | | | | | |
| | Carboxyvinyl polymer | | | | | | | 0.5 | 0.5 | 0.5 | 0.5 | 0.5 |
| Evaluation | Detergency | Wet condition | C | C | C | C | C | C | C | C | C | C |
| | | Non-wet condition | F | C | C | C | C | C | C | C | C | C |
| | Feel of use | | C | F | F | F | F | C | C | F | F | C |

Tables 2 and 3 demonstrates that the cleansing cosmetics according to the embodiment of the present invention are readily miscible with a makeup stain, regardless of whether the skin is wet or not, can exhibit excellent detergency, and can be washed away cleanly without leaving an oily feel by rinsing with water.

Industrial Applicability

The polyglycerol di-(alkyl/alkenyl) ethers according to the embodiment of the present invention offer an extremely excellent surface activity, can thereby exhibit a sufficient surface activity even in an amount smaller than that of a conventional equivalent, and can contribute to extreme reduction of environmental load and skin irritation. Cleansing cosmetic compositions including the polyglycerol di-(alkyl/alkenyl) ethers according to the embodiment of the present invention offer a light feel of use without stickiness. The cleansing cosmetic compositions are readily miscible with a makeup stain, regardless of whether the skin is wet or not, can exhibit excellent detergency, can be washed away cleanly without leaving an oily feel by rinsing with water, and are advantageously usable as cleansing agents for makeup cosmetics.

## Claims

1. A polyglycerol di-(alkyl/alkenyl) ether represented by Formula (1) expressed as follows:

[Chem. 1]

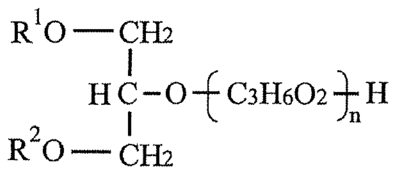

(1)

where $R^1$ and $R^2$ are identical or different and are independently one selected from a straight- or branched-chain alkyl group and a straight- or branched-chain alkenyl group, where the alkyl group may have one or more hydroxyl groups; and n indicates a number of glycerol units and is an integer of 2 or more.

2. The polyglycerol di-(alkyl/alkenyl) ether according to claim 1, wherein $R^1$ and $R^2$ are identical or different and are independently one selected from a straight- or branched-chain alkyl group having 6 to 22 carbon atoms and a straight- or branched-chain alkenyl group having 6 to 22 carbon atoms, where the alkyl group may have one or more hydroxyl groups; and n is an integer of from 2 to 25.

3. A cosmetic composition comprising the polyglycerol di-(alkyl/alkenyl) ether of one of claims 1 and 2.

4. A cleansing cosmetic composition comprising:

    1 to 70 percent by weight of the polyglycerol di-(alkyl/alkenyl) ether of one of claims 1 and 2; and
    30 to 99 percent by weight of water.

5. A cleansing cosmetic comprising the cleansing cosmetic composition of claim 4.

6. The cleansing cosmetic according to claim 5, further comprising a polymer compound.

[Fig. 1]

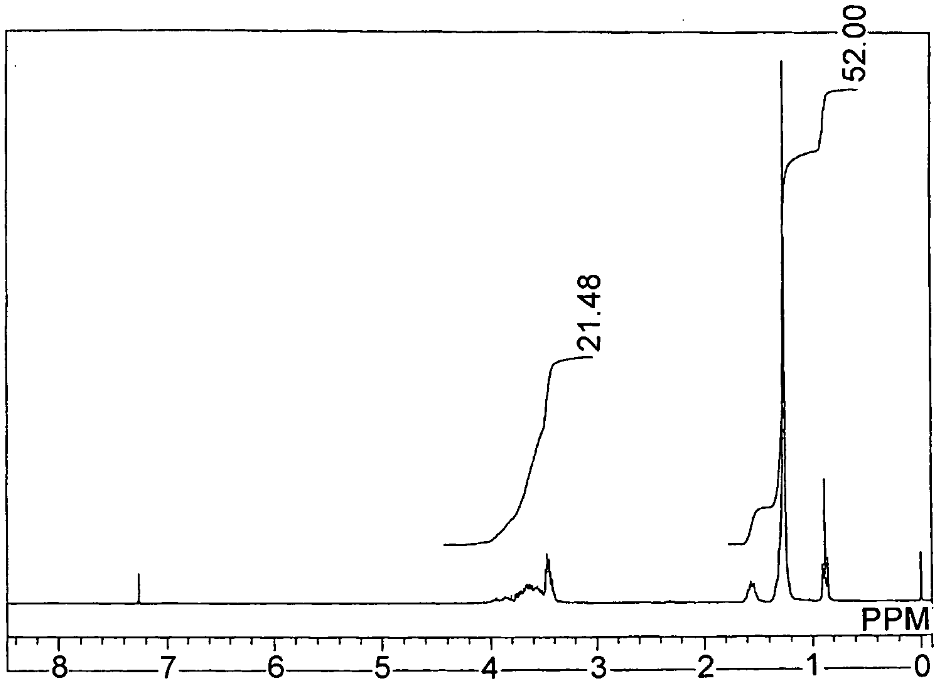


[Fig. 2]

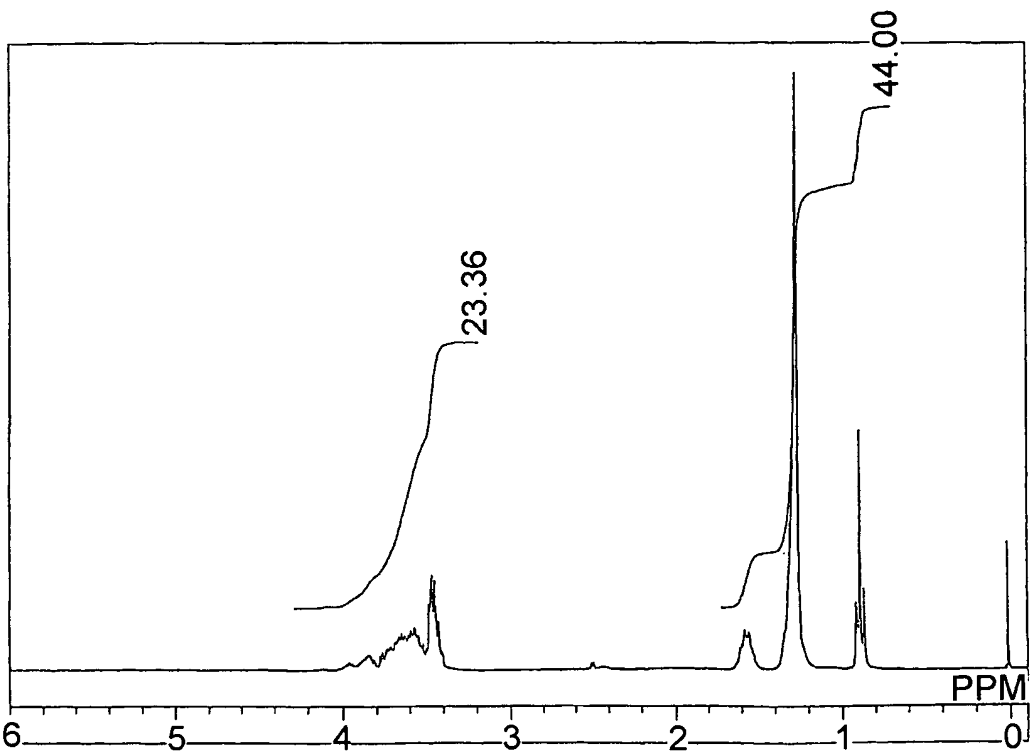

[Fig. 3]

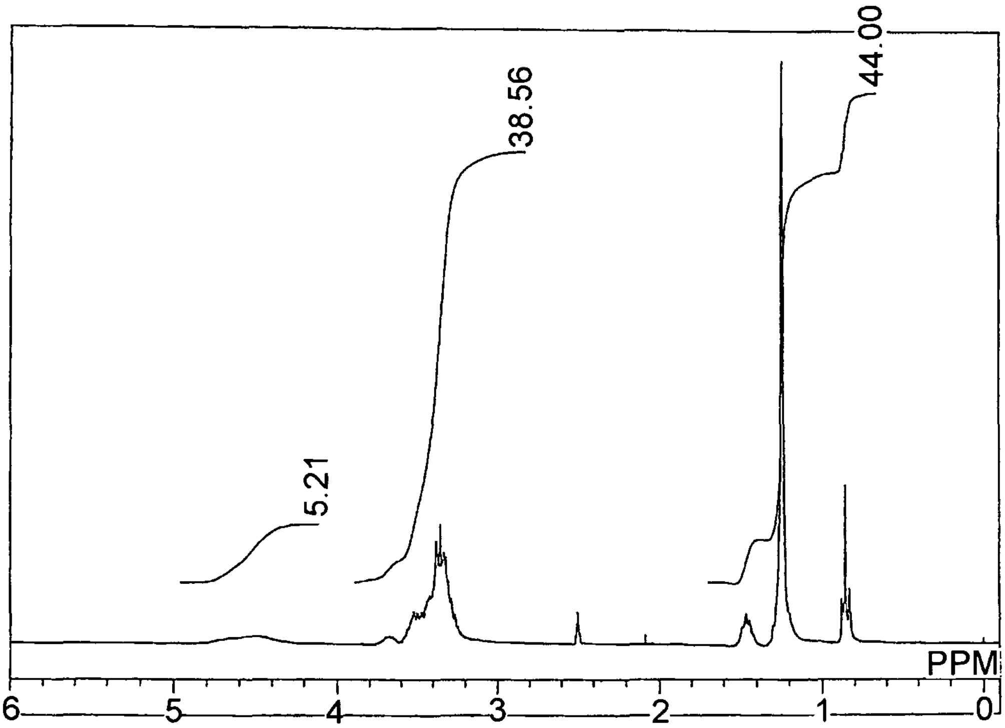


[Fig. 4]

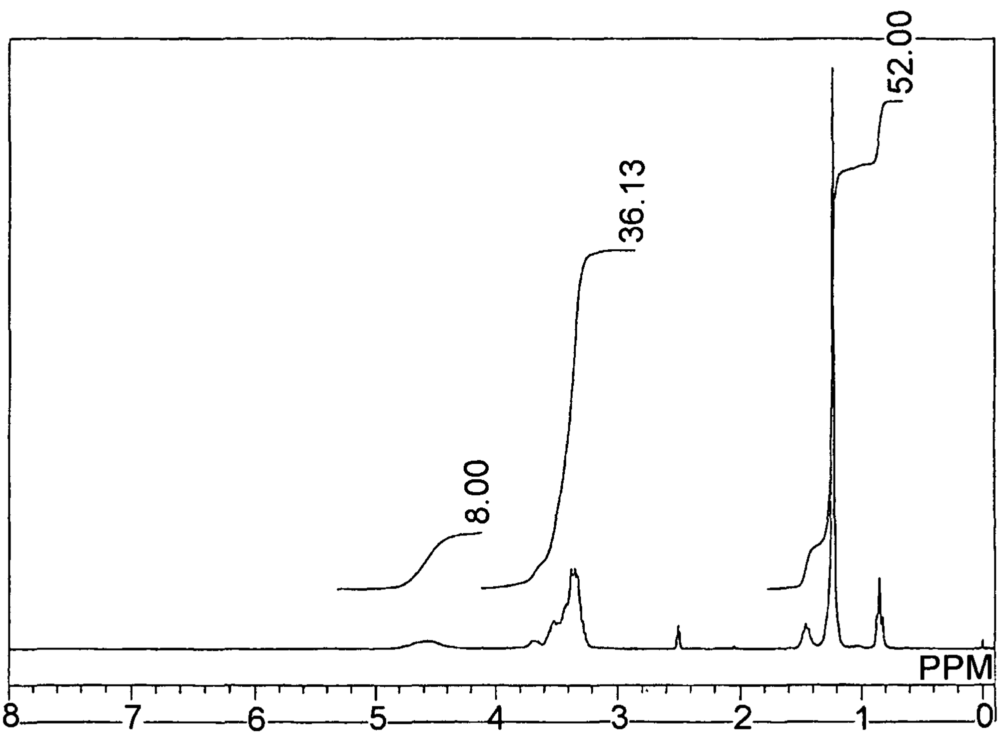

**INTERNATIONAL SEARCH REPORT**

International application No. PCT/JP2013/052461

A. CLASSIFICATION OF SUBJECT MATTER
*C07C43/13*(2006.01)i, *A61K8/34*(2006.01)i, *A61Q1/14*(2006.01)i, *C07C43/178*(2006.01)i, *C08G65/28*(2006.01)i

According to International Patent Classification (IPC) or to both national classification and IPC

B. FIELDS SEARCHED

Minimum documentation searched (classification system followed by classification symbols)
C07C43/13, A61K8/34, A61Q1/14, C07C43/178, C08G65/28

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
JSTPlus/JMEDPlus/JST7580(JDreamII), JCHEM(JDreamII), CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2000-239208 A (Taiyo Kagaku Co., Ltd.),<br>05 September 2000 (05.09.2000),<br>claims; paragraphs [0005], [0013]<br>& CA 2356474 A & JP 2001-064236 A<br>& JP 2001-114720 A & US 2002/0035238 A1 | 1,3-6<br>2 |
| Y | JP 56-63936 A (L'Oreal),<br>30 May 1981 (30.05.1981),<br>claims; page 4, upper left column, 3rd paragraph; pages 4 to 8, examples 1 to 9; page 10, example A5<br>& BE 885720 A1 & CA 1167070 A1<br>& CH 646129 A5 & DE 3039008 A1<br>& FR 2467838 A1 & GB 2060665 A<br>& IT 1129314 B & US 4399313 A<br>& US 4465860 A & US 5071640 A | 1-6 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 April, 2013 (05.04.13) | 16 April, 2013 (16.04.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No. PCT/JP2013/052461

C (Continuation). DOCUMENTS CONSIDERED TO BE RELEVANT

| Category* | Citation of document, with indication, where appropriate, of the relevant passages | Relevant to claim No. |
|---|---|---|
| Y | JP 54-44039 A (Nihon Surfactant Kogyo Kabushiki Kaisha), 07 April 1979 (07.04.1979), claims; pages 2 to 3, synthesis examples 1 to 6 (Family: none) | 1-6 |
| Y | JP 2010-526778 A (Dow Global Technologies Inc.), 05 August 2010 (05.08.2010), claims; paragraph [0034] & CA 2685319 A1 & CN 101668728 A & EP 2152653 A1 & MX 2009011606 A & RU 2009143873 A & WO 2008/134389 A1 | 1-6 |
| Y | WO 2007/114061 A1 (Kao Corp.), 11 October 2007 (11.10.2007), paragraph [0002] & CN 101400635 A & EP 2003110 A2 & JP 2007-269730 A & US 2010/0234646 A1 | 1-6 |
| Y | JP 59-93022 A (Kao Soap Co., Ltd.), 29 May 1984 (29.05.1984), claims; pages 11 to 24, examples 1 to 35 & DE 3341366 A1 & FR 2535969 A1 & GB 2130206 A & JP 59-175445 A & US 4543258 A | 1-6 |
| X<br>A | JP 60-224638 A (Kao Soap Co., Ltd.), 09 November 1985 (09.11.1985), claims; page 6, lower right column, 2nd paragraph; pages 7 to 8, product 1 & EP 162239 A1 & US 4859696 A & US 4948588 A | 1-3<br>4-6 |
| A | JP 2007-63338 A (Dai-Ichi Kogyo Seiyaku Co., Ltd.), 15 March 2007 (15.03.2007), claims; paragraphs [0037] to [0046] & CN 1923858 A & EP 1760095 A2 & JP 2007-091914 A & JP 2007-092010 A & JP 2007-131732 A & KR 10-2007-0026061 A & US 2007/0049687 A1 | 1-6 |
| A | M.WEINHART, et al., Linear Poly(methyl glcerol) and Linear Polyglycerol as Potent Protein and Cell Resistant Alternatives to Poly(ethylene glycol), Chemistry-An Asian Journal, Vol.5, No.9, p.1992-2000 (2010). | 1-6 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H08283123 A
- JP S59105073 A
- JP S649304 A
- JP 2001354998 A


### Non-patent literature cited in the description


- **RYOHEI ODA ; KAZUHIRO TERAMURA.** Kaimen-kasseizai no Gosei to sono Oyo. Maki Shoten, March 1957